(19) Europäisches Patentamt
European Patent Office
Office européen des brevets

(11) **EP 4 566 521 B1**

(12) **EUROPEAN PATENT SPECIFICATION**

(45) Date of publication and mention
of the grant of the patent:
**01.04.2026 Bulletin 2026/14**

(21) Application number: **24214836.9**

(22) Date of filing: **22.11.2024**

(51) International Patent Classification (IPC):
*A61B 5/0205* (2006.01)     *A61B 5/16* (2006.01)
*B60W 40/08* (2012.01)     *A61B 5/318* (2021.01)
*A61B 5/18* (2006.01)     *A61B 5/00* (2006.01)
*B60W 40/00* (2006.01)     *B60W 50/08* (2020.01)
*A61M 21/00* (2006.01)     *B60W 50/14* (2020.01)

(52) Cooperative Patent Classification (CPC):
**A61B 5/18; A61B 5/0205; A61B 5/165;**
**A61B 5/4809; A61B 5/6893; B60W 40/08;**
**B60W 50/082; B60W 50/14;** A61B 5/318;
A61B 5/4266; A61B 5/4836; A61B 5/7405;
A61B 2503/22; B60W 2040/0872; B60W 2540/10;
(Cont.)

(54) **VEHICLE CONTROL SYSTEM**

FAHRZEUGSTEUERUNGSSYSTEM

SYSTÈME DE COMMANDE DE VÉHICULE

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**

(30) Priority: **04.12.2023 JP 2023204546**

(43) Date of publication of application:
**11.06.2025 Bulletin 2025/24**

(73) Proprietor: **TOYOTA JIDOSHA KABUSHIKI**
**KAISHA**
**Aichi-ken, 471-8571 (JP)**

(72) Inventor: **HARA, Yoshikazu**
**Toyota-Shi**
**Aichi-Ken 471-8571 (JP)**

(74) Representative: **Cabinet Beau de Loménie**
**103, rue de Grenelle**
**75340 Paris Cedex 07 (FR)**

(56) References cited:
JP-A- 2023 077 193     US-A1- 2020 383 580
US-A1- 2021 229 550     US-A1- 2022 041 070

**EP 4 566 521 B1**

(52) Cooperative Patent Classification (CPC): (Cont.)
B60W 2540/14; B60W 2540/16; B60W 2540/22;
B60W 2540/221

2

## Description

### 1. Field of the Invention

The present disclosure relates to a vehicle control system.

### 2. Description of Related Art

Japanese Patent No. 6787507 discloses a battery electric vehicle that can reproduce the manual gear shifting behavior of a manual transmission vehicle (MT vehicle) in a pseudo manner.
JP 2023 077193 A deals with a user approach answering device
US 2020/383580 A1 deals with apparatus, system, and method for physiological sensing in vehicles.
US 2022/041070 A1 and US 2021/229550 A1 deal with an electric vehicle.

### SUMMARY OF THE INVENTION

Mental fatigue in a vehicle driver is undesirable from a standpoint of appropriate driving. A technology that allows the driver to drive the vehicle more comfortably is desired.

A first aspect is defined in claim 1, and relates to a vehicle control system that controls a vehicle. The vehicle control system includes a single or plurality of processors. The single or plurality of processors acquires a mental fatigue degree of a driver of the vehicle using at least a sensor that is equipped in the vehicle. The single or plurality of processors outputs a relaxation sound through a speaker that is equipped in the vehicle, when an actuation condition is satisfied, the relaxation sound being a sound for reducing the mental fatigue degree, the actuation condition including at least a condition that the mental fatigue degree exceeds a first threshold.

In the vehicle control system according to the first aspect of the present disclosure, the actuation condition may further include a condition that the driver permits the output of the relaxation sound.

In the vehicle control system according to the first aspect of the present disclosure, the sensor may include at least one of an electrocardiograph and a sweating sensor, the electrocardiograph and the sweating sensor being equipped in a device that is used by the driver at a time of driving operation or in a wearable terminal that is worn by the driver. The single or plurality of processors may be configured to acquire the mental fatigue degree, based on at least one of data about heartbeat of the driver that is detected by the electrocardiograph and the sweating amount of the driver that is detected by the sweating sensor.

In the vehicle control system according to the first aspect of the present disclosure, the vehicle is a battery electric vehicle configured to use an electric motor as a dynamic power device for traveling, and includes a pseudo clutch pedal and a pseudo shift device, the pseudo clutch pedal is operated at a time of operation of the pseudo shift device, a driving mode of the battery electric vehicle includes a three-pedal mode in which the output of the electric motor with respect to operation of an accelerator pedal is changed depending on the operation of the pseudo clutch pedal and the operation of the pseudo shift device, and in the three-pedal mode, the single or plurality of processors is configured to acquire the mental fatigue degree, and is configured to output the relaxation sound when the actuation condition is satisfied.

In the vehicle control system according to the first aspect of the present disclosure, the single or plurality of processors may output a sound in nature as the relaxation sound through the speaker.

In the vehicle control system according to the first aspect of the present disclosure, the sensor may be equipped in the pseudo shift device.

In the vehicle control system according to the first aspect of the present disclosure, the driving mode of the battery electric vehicle may further include a two-pedal mode in which the operation of the pseudo clutch pedal is not necessary, and the single or plurality of processors may be further configured to switch the driving mode from the three-pedal mode to the two-pedal mode, when a mode switching condition is satisfied, the mode switching condition including at least a condition that the mental fatigue degree exceeds a second threshold during the three-pedal mode.

In the vehicle control system according to the first aspect of the present disclosure, the mode switching condition may further include a condition that the driver permits the switching from the three-pedal mode to the two-pedal mode.

In the vehicle control system according to the first aspect of the present disclosure, the single or plurality of processors may be configured to determine whether the mode switching condition is satisfied, after the actuation condition is satisfied and the relaxation sound is output.

In the first aspect, when the actuation condition including at least the condition that the mental fatigue degree of the driver exceeds the threshold is satisfied, the relaxation sound that reduces the mental fatigue degree is output from the speaker that is equipped in the vehicle. Thereby, the mental fatigue of the driver is reduced. As a result, the driver can more comfortably drive the vehicle.

### BRIEF DESCRIPTION OF THE DRAWINGS

Features, advantages, and technical and industrial significance of exemplary embodiments of the invention will be described below with reference to the accompanying drawings, in which like signs denote like elements, and wherein:

FIG. 1 is a conceptual diagram showing a vehicle and a vehicle control system;

FIG. 2 is a diagram showing exemplary sensors;

FIG. 3A is a diagram showing data about a heartbeat interval (R-R interval (RRI)) that indicates a time difference between a generation time of an R-wave and a generation time of a next R-wave in heartbeat data about a driver;

FIG. 3B is a diagram showing a Lorenz plot in which an n-th value (RRI(n)) of R-R intervals is plotted on an abscissa axis (x-axis) and an n+1-th value (RRI(n+1)) subsequent to the n-th value is plotted on an ordinate axis (y-axis);

FIG. 3C is a graph showing the sweating amount of the driver;

FIG. 4 is a block diagram showing an exemplary functional configuration of the vehicle control system according to a first embodiment;

FIG. 5 is a flowchart showing an exemplary process by the vehicle control system according to the first embodiment;

FIG. 6 is a conceptual diagram for describing an outline of a second embodiment;

FIG. 7 is a flowchart showing an exemplary process by a vehicle control system according to the second embodiment;

FIG. 8 is a conceptual diagram for describing an outline of a third embodiment;

FIG. 9 is a block diagram showing an exemplary functional configuration of a vehicle control system according to the third embodiment;

FIG. 10 is a flowchart showing an exemplary process by the vehicle control system according to the third embodiment;

FIG. 11 is a block diagram showing an exemplary functional configuration of a vehicle control system according to a fourth embodiment;

FIG. 12 is a flowchart showing an exemplary process by the vehicle control system according to the fourth embodiment;

FIG. 13 is a flowchart showing another exemplary process by the vehicle control system according to the fourth embodiment;

FIG. 14 is a block diagram showing a first exemplary configuration of a dynamic power control system of a battery electric vehicle:

FIG. 15 is a diagram showing each example of an engine model, a clutch mode and a transmission model that constitute an MT vehicle model;

FIG. 16 is a diagram showing a torque characteristic of an electric motor that is realized by a motor control using the MT vehicle model; and

FIG. 17 is a block diagram showing a second exemplary configuration of the dynamic power control system of the battery electric vehicle.

DETAILED DESCRIPTION OF EMBODIMENTS

Embodiments of the present disclosure will be described with reference to the accompanying drawings.

1. First Embodiment

1-1. Outline

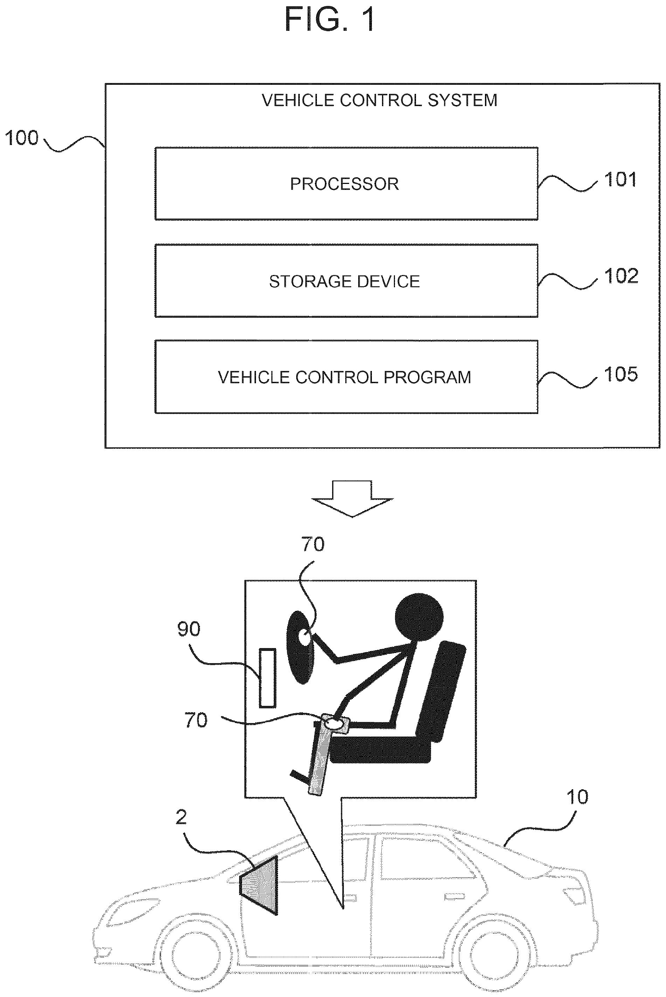

FIG. 1 is a conceptual diagram showing a vehicle 10 and a vehicle control system 100 according to an embodiment. The vehicle 10 may be an engine vehicle in which an internal combustion engine is used as a dynamic power device for traveling, or may be a battery electric vehicle in which an electric motor is used as a dynamic power device for traveling. The vehicle 10 may be a manual transmission vehicle (MT vehicle).

The vehicle control system 100 controls the vehicle 10. The whole of the vehicle control system 100 may be equipped in the vehicle 10. As another example, at least a part of the vehicle control system 100 may be included in a management server that can communicate with the vehicle 10. That is, the vehicle control system 100 may remotely control the vehicle 10. The vehicle control system 100 may be distributed between the vehicle 10 and the management server.

In general, the vehicle control system 100 includes a single or plurality of processors 101 (referred to as merely a processor 101, hereinafter) and a single or plurality of storage devices 102 (referred to as merely a storage device 102, hereinafter). The processor 101 executes various processes. Examples of the processor 101 include a general-purpose processor, a specific-use processor, a central processing unit (CPU), a graphics processing unit (GPU), an application specific integrated circuit (ASIC), a field-programmable gate array (FPGA), an integrated circuit, a conventional circuit, and/or combinations of them. The processor 101 can be also referred to as a circuitry or a processing circuitry. The circuitry is hardware that has a program for realizing written functions, or is hardware that executes functions. The storage device 102 stores (contains) a variety of information. Examples of the storage device 102 include a volatile memory, a non-volatile memory, a hard disk drive (HDD), and a solid state drive (SSD). Functions of the vehicle control system 100 are realized by the cooperation between the processor 101 and the storage device 102.

A single or plurality of vehicle control programs 105 (referred to as merely a vehicle control program 105, hereinafter) is a computer program that is executed by the processor 101. Functions of the vehicle control system 100 may be realized by the cooperation between the processor 101 that executes the vehicle control program 105 and the storage device 102. The vehicle control program 105 is contained in the storage device 102. Alternatively, the vehicle control program 105 may be recorded in a computer-readable recording medium.

A driver of the vehicle 10 sometimes has a feeling of mental fatigue. For example, in the case where another vehicle cuts in front of the vehicle 10 or in the case where the advancing speed of the vehicle 10 is low due to congestion, the driver can get stressed. As another example, in the case where the vehicle 10 is an MT vehicle, the long-time operation of a clutch pedal requires the driver to maintain concentration power and attention for a long time, and can give stress to the driver. The mental fatigue of the driver is undesirable from a standpoint of appropriate driving. A technology that allows the driver to drive the vehicle 10 more comfortably is desired.

Hence, in the embodiment, a sensor 70 for detecting the mental fatigue of the driver is equipped in the vehicle 10. Furthermore, a speaker 2 for outputting a relaxation sound that reduces the mental fatigue of the driver is equipped in the vehicle 10. The relaxation sound is contained in the storage device 102. Details of the relaxation sound will be described later.

The vehicle control system 100 (processor 101) acquires a mental fatigue degree M of the driver, using the sensor 70 that is equipped in the vehicle 10. The mental fatigue degree M quantitatively shows the degree of the mental fatigue of the driver. An actuation condition for outputting the relaxation sound includes at least a condition that the mental fatigue degree M of the driver exceeds a first threshold Mth1. The vehicle control system 100 (processor 101) determines whether the actuation condition is satisfied, based on at least the mental fatigue degree M. In the case where the actuation condition is satisfied, the vehicle control system 100 (processor 101) outputs the relaxation sound that reduce the mental fatigue degree, through the speaker 2 that is equipped in the vehicle 10. Thereby, the mental fatigue of the driver is reduced. As a result, the driver can more comfortably drive the vehicle 10.

1-2. Example of Sensor

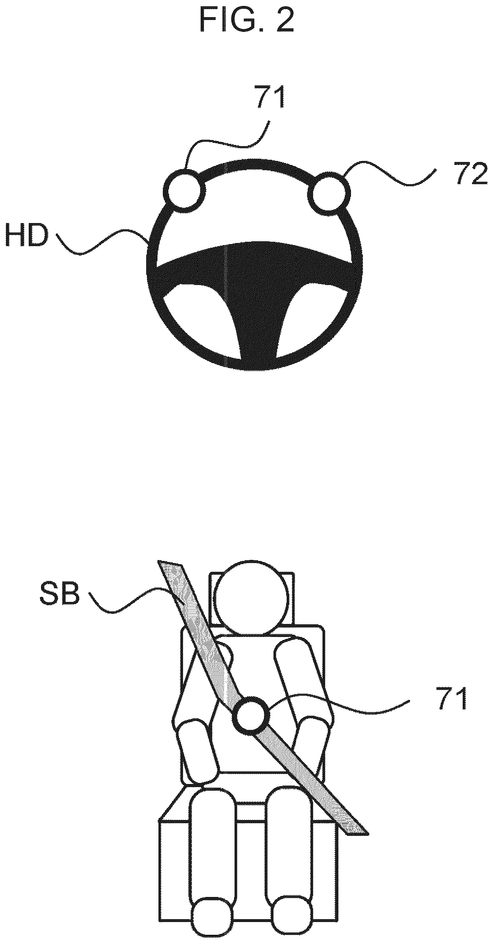

FIG. 2 is a diagram showing examples of the sensor 70. The sensor 70 includes at least one of one or more electrocardiographs 71 that detect heartbeat data about the driver and one or more sweating sensors 72 that detect the sweating amount of the driver. For example, the electrocardiograph 71 is installed at a position that allows the detection of the heartbeat of the driver, that is, at a device (e.g.: a steering wheel HD and a seatbelt SB) that is used by the driver at the time of driving operation. For example, the sweating sensor 72 is installed at a position where the palm of the hand of the driver contacts, that is, at a device (e.g.: the steering wheel HD) that is used by the driver at the time of driving operation. The sweating amount of the driver may be detected by the sweating sensor 72 equipped in a wearable terminal (e.g.: a smartwatch) that is worn by the driver.

The vehicle control system 100 acquires the mental fatigue degree M based on at least one of the heartbeat data about the driver that is detected by the electrocardiograph 71 and the sweating amount of the driver that is detected by the sweating sensor 72.

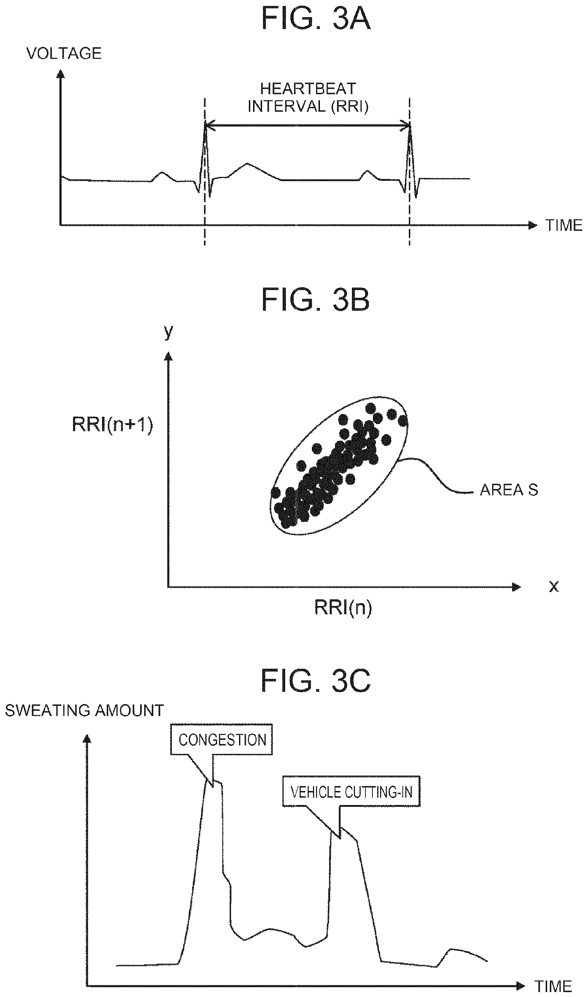

An exemplary calculation of the mental fatigue degree M based on the heartbeat data about the driver will be discussed. As shown in FIG. 3A, the heartbeat data about the driver includes data about a heartbeat interval (R-R interval (RRI)) that indicates a time difference between a generation time of an R-wave and a generation time of a next R-wave. For example, the mental fatigue degree M is calculated based on the magnitude of an area S of a Lorenz plot that is generated by the data about the heartbeat interval (R-R interval) of the driver.

As shown in FIG. 3B, in the Lorenz plot, an n-th value (RRI(n)) of R-R intervals is plotted on an abscissa axis (x-axis), and an n+1-th value (RRI(n+1)) subsequent to the n-th value is plotted on an ordinate axis (y-axis). That is, a point $(x, y) = (RRI(n), RRI(n+1))$ that are constituted by two consecutive R-R intervals is plotted on the graph. The area S of the Lorenz plot is calculated by the ellipse approximation of the region of an aggregation of plots. The area S of the Lorenz plot indicates a spread range of the plots. The area S of the Lorenz plot is an index that indicates whether the heartbeat of the driver is stable or unstable. It is known that a state where the area S of the Lorenz plot is large is a relaxed state and a state where the area S of the Lorenz plot is small is a stressed state. Consequently, the mental fatigue degree M can be calculated based on the magnitude of the area S of the Lorenz plot. For example, the mental fatigue degree M is calculated to a higher value as the area S of the Lorenz plot is smaller.

An exemplary calculation of the mental fatigue degree M based on the sweating amount of the driver will be discussed. For example, the sweating amount of the driver is expressed by a graph shown in FIG. 3C. As shown in FIG. 3C, for example, in the case where the vehicle 10 is caught in congestion or in the case where another vehicle suddenly cuts in front of the vehicle 10, the sweating amount increases. Accordingly, the mental fatigue degree M changes depending on the sweating amount of the driver. As the sweating amount of the driver is larger, the mental fatigue degree M is higher. The sweating amount of the driver may be used as the mental fatigue degree M with no change.

Other than the above methods, the mental fatigue degree M may be estimated based on a recognition result about the face (expression) of the driver by a camera that is equipped in the vehicle 10.

In the case where the actuation condition including at least the condition that the mental fatigue degree M exceeds the first threshold Mth1 is satisfied, the vehicle control system 100 outputs the relaxation sound that reduces the mental fatigue degree M of the driver.

The relaxation sound is a sound for reducing the mental fatigue degree M. Specifically, the relaxation sound is a sound that increases the area S of the Lorenz plot. Alternatively, the relaxation sound is a sound that gives a relaxation feeling to the driver for decreasing the sweating amount. Examples of the relaxation sound include a sound in nature, as exemplified by a murmuring sound of streams. The relaxation sound may be previously selected. By outputting the relaxation sound, a relaxation feeling can be given to the driver, so that the mental fatigue of the driver is reduced. As a result, the driver can more comfortably drive the vehicle 10.

1-3. Exemplary Functional Configuration and Exemplary Process

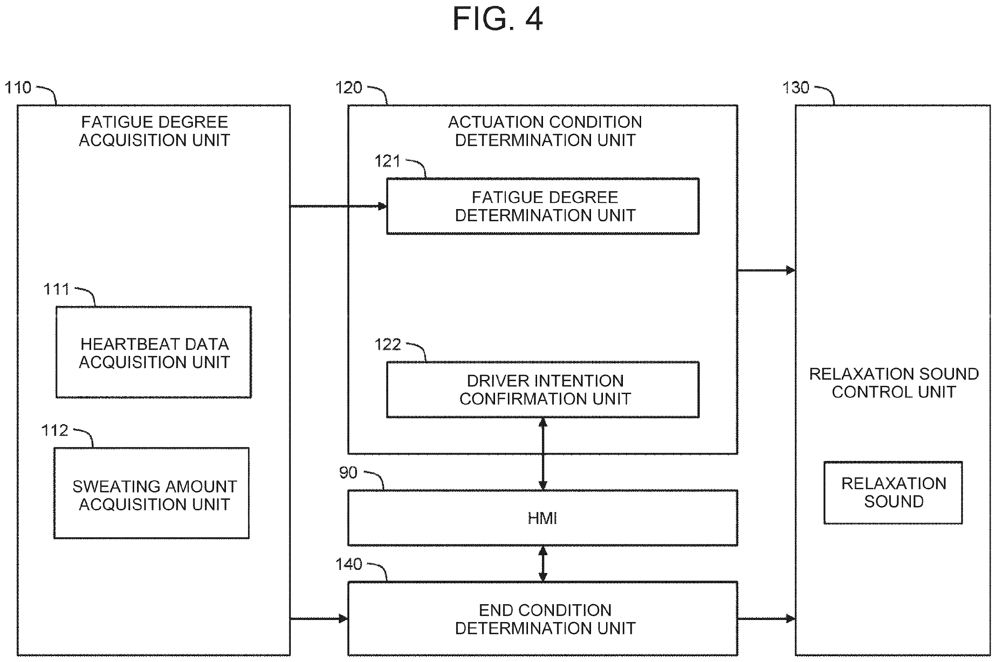

FIG. 4 is a block diagram showing an exemplary functional configuration of the vehicle control system 100 according to a first embodiment. The vehicle control system 100 includes a fatigue degree acquisition unit 110, an actuation condition determination unit 120, a relaxation sound control unit 130, and an end condition determination unit 140, as functional blocks. The functional blocks may be realized by the cooperation between the processor 101 that executes the vehicle control program 105 and the storage device 102. Some of the functional blocks may be included in the management server that can communicate with the vehicle 10.

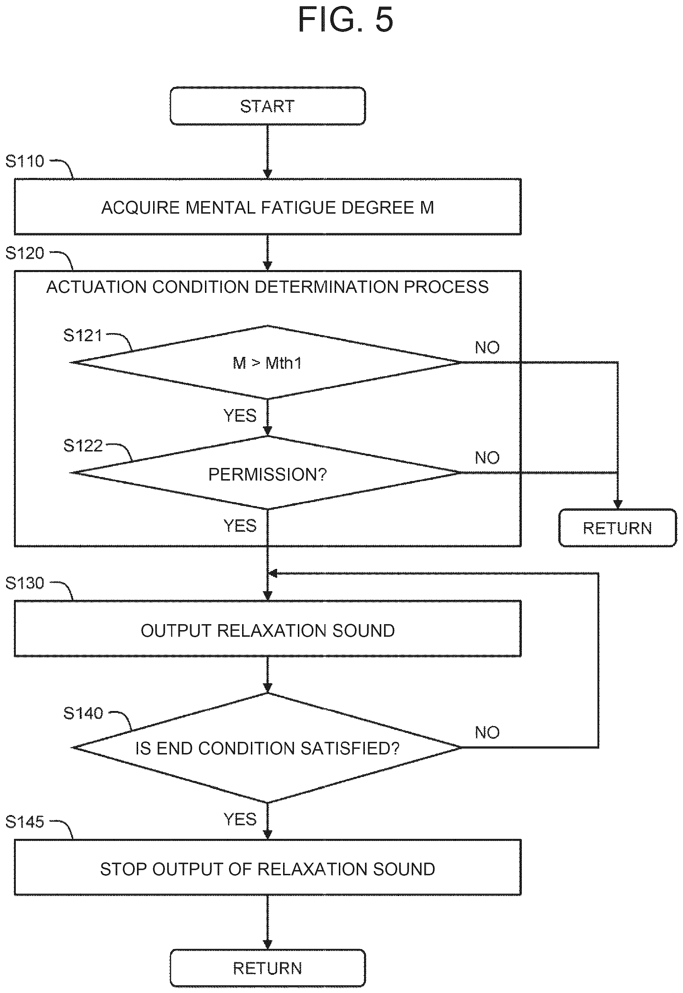

FIG. 5 is a flowchart showing an exemplary process by the vehicle control system 100 according to the first embodiment. With reference to FIG. 4 and FIG. 5, the exemplary process by the vehicle control system 100 will be described below.

In step S110, the fatigue degree acquisition unit 110 acquires sensor detection information indicating the detection result of the sensor 70 that is equipped in the vehicle 10. In the case where the fatigue degree acquisition unit 110 is included in the management server, the fatigue degree acquisition unit 110 communicates with the vehicle 10, and acquires the sensor detection information. The fatigue degree acquisition unit 110 acquires the mental fatigue degree M of the driver, based on the sensor detection information.

For example, the fatigue degree acquisition unit 110 includes a heartbeat data acquisition unit 111 and a sweating amount acquisition unit 112. The heartbeat data acquisition unit 111 acquires the heartbeat data about the driver that is detected by the electrocardiograph 71 (see FIG. 2). The sweating amount acquisition unit 112 acquires the sweating amount of the driver that is detected by the sweating sensor 72 (see FIG. 2). The heartbeat data and sweating amount about the driver correspond to the sensor detection information. Moreover, the fatigue degree acquisition unit 110 acquires the mental fatigue degree M based on at least one of the heartbeat data and sweating amount about the driver. In this case, the mental fatigue degree M is higher, as the area S of the Lorenz plot that is generated based on the heartbeat data about the driver is smaller or as the sweating amount of the driver is larger. The sweating amount of the driver may be used as the mental fatigue degree M with no change.

In step S120, the actuation condition determination unit 120 determines whether a predetermined actuation condition is satisfied. In the case where the predetermined actuation condition is not satisfied (step S120; No), the process in this cycle ends. On the other hand, in the case where the predetermined actuation condition is satisfied (step S120; Yes), the process proceeds to step S130.

In the example shown in FIG. 4 and FIG. 5, the predetermined actuation condition includes a first condition and a second condition. The first condition is that the mental fatigue degree M exceeds the first threshold Mth1. The second condition is that the driver permits the output of the relaxation sound. The actuation condition determination unit 120 includes a fatigue degree determination unit 121 and a driver intention confirmation unit 122, for determining whether the first condition and the second condition are satisfied, respectively. At least one of the fatigue degree determination unit 121 and the driver intention confirmation unit 122 may be included in the management server that can communicate with the vehicle 10.

In step S121, the fatigue degree determination unit 121 determines whether the mental fatigue degree M is higher than the first threshold Mth1, that is, whether the first condition is satisfied. In the case where the first condition is not satisfied (step S121; No), the actuation condition is not satisfied. On the other hand, in the case where the first condition is satisfied (step S121; Yes), the process proceeds to step S122.

In step S122, the driver intention confirmation unit 122 determines whether the driver has permitted the output of the relaxation sound, that is, whether the second condition is satisfied. More specifically, the vehicle 10 is equipped with a human-machine interface (HMI) 90 (see FIG. 1). The HMI 90 includes an output device and an input device. Examples of the output device include a touch panel, a display, and a speaker. Examples of the input device include a touch panel and a button. The driver intention confirmation unit 122 inquires of the driver whether to agree to the output of the relaxation sound, through the output device of the HMI 90. An inquiry message may be displayed on the display, may be given from the speaker, or may be output from both the display and the speaker. In response to the inquiry message, the driver inputs "permission" or "rejection", using the input device of the HMI 90. The driver intention confirmation unit 122 can determine whether the second condition is satisfied, based on the input from the driver. In the case where the second condition is not satisfied (step S122; No), the actuation condition is not satisfied. On the other hand, in the case where the second condition is satisfied (step S122; Yes), the actuation condition is satisfied (step S120; Yes), and the process proceeds to step S130.

In step S130, the relaxation sound control unit 130 acquires the relaxation sound contained in the storage device 102, and outputs the relaxation sound through the speaker 2. When the relaxation sound is output, the relaxation sound control unit 130 may notify the driver that the relaxation sound is output, through the output device of the HMI 90.

In step S140, the end condition determination unit 140 determines whether an end condition is satisfied. For example, the end condition is that the mental fatigue degree M of the driver becomes equal to or lower than the first threshold Mth1. As anther example, the end condition may be that a certain time elapses from the start of the output of the relaxation sound. Furthermore, as another example, the end condition may be that the driver gives an instruction to stop the output of the relaxation sound through the HMI 90. In the case where the end condition is not satisfied (step S140; No), the process returns to step S130, and the output of the relaxation sound is continued. On the other hand, in the case where the end condition is satisfied (step S140; Yes), the process proceeds to step S145.

In step S145, the relaxation sound control unit 130 stops the output of the relaxation sound.

1-4. Modification

The predetermined actuation condition may exclude the second condition. In that case, step S122 is skipped.

2. Second Embodiment

2-1. Outline

In the case where the operation of the clutch pedal is necessary, stress can be given to the driver. The stress due to the operation of the clutch pedal is a kind of the mental fatigue of the driver. A second embodiment proposes a technology that makes it possible to reduce the stress due to the operation of the clutch pedal.

For example, a vehicle 10 assumed in the second embodiment is a manual transmission vehicle (MT vehicle) that includes the clutch pedal. As another example, the vehicle 10 may be a battery electric vehicle that can reproduce the manual gear shifting behavior of the MT vehicle in a pseudo manner (see Japanese Patent No. 6787507). A case where the vehicle 10 is a battery electric vehicle that can reproduce the manual gear shifting behavior of the MT vehicle in a pseudo manner will be discussed below. The same goes for a case where the vehicle 10 is an ordinary MT vehicle.

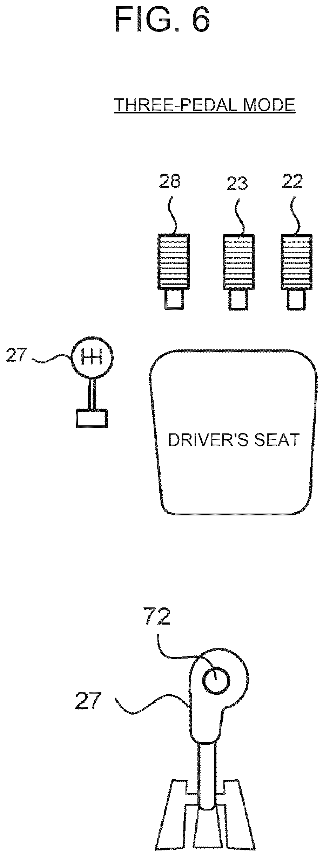

FIG. 6 is a conceptual diagram for describing an outline of the second embodiment. The vehicle 10 includes an accelerator pedal 22, a brake pedal 23, a pseudo clutch pedal 28, and a pseudo shift lever 27 (pseudo shift device).

The pseudo shift lever 27 has a structure that simulates a shift lever included in the MT vehicle. The disposition and operational feeling of the pseudo shift lever 27 are equivalent to those in the actual MT vehicle.

For the pseudo shift lever 27, for example, positions corresponding to gear steps: first gear, second gear, third gear, forth gear, fifth gear, sixth gear, reverse, and neutral are provided.

The pseudo clutch pedal 28 has a structure that simulates the clutch pedal included in the MT vehicle. The disposition and operational feeling of the pseudo clutch pedal 28 are equivalent to those in the actual MT vehicle. The pseudo clutch pedal 28 is operated at the time of the operation of the pseudo shift lever 27. That is, when the driver wants to perform the setting alteration of the gear step by the pseudo shift lever 27, the driver depresses the pseudo clutch pedal 28. When the setting alteration of the gear step finishes, the driver terminates the depressing, and returns the pseudo clutch pedal 28 to the original position.

The driving mode of the vehicle 10 (battery electric vehicle) includes a "three-pedal mode" in which the manual gear shifting behavior and driving characteristic of the MT vehicle are simulated. In the three-pedal mode, the output of the electric motor with respect to the operation of the accelerator pedal 22 is changed depending on the operation of the pseudo clutch pedal 28 and the operation of the pseudo shift lever 27. A method for realizing the three-pedal mode in the battery electric vehicle will be described later in Section 6 in detail.

In the three-pedal mode, the vehicle control system 100 acquires the mental fatigue degree M of the driver, using the sensor 70 that is equipped in the vehicle 10. For example, the sensor 70 includes the electrocardiograph 71 and the sweating sensor 72. The sweating sensor 72 may be equipped in the pseudo shift lever 27 that is operated by the driver in the three-pedal mode. In this case, the vehicle control system 100 can acquire the mental fatigue degree M, based on the sweating amount of the driver that is detected by the sweating sensor 72 equipped in the pseudo shift lever 27.

The actuation condition for outputting the relaxation sound includes at least the condition that the mental fatigue degree M of the driver exceeds the first threshold Mth1. In the case where the actuation condition is satisfied, the vehicle control system 100 (processor 101) outputs the relaxation sound through the speaker 2.

By outputting the relaxation sound, the driver's stress due to the operation of the pseudo clutch pedal 28 is reduced. As a result, the driver can more comfortably drive the vehicle 10. Particularly, the driver can comfortably drive the vehicle 10 while enjoying the three-pedal mode.

2-2. Exemplary Functional Configuration and Exemplary Process

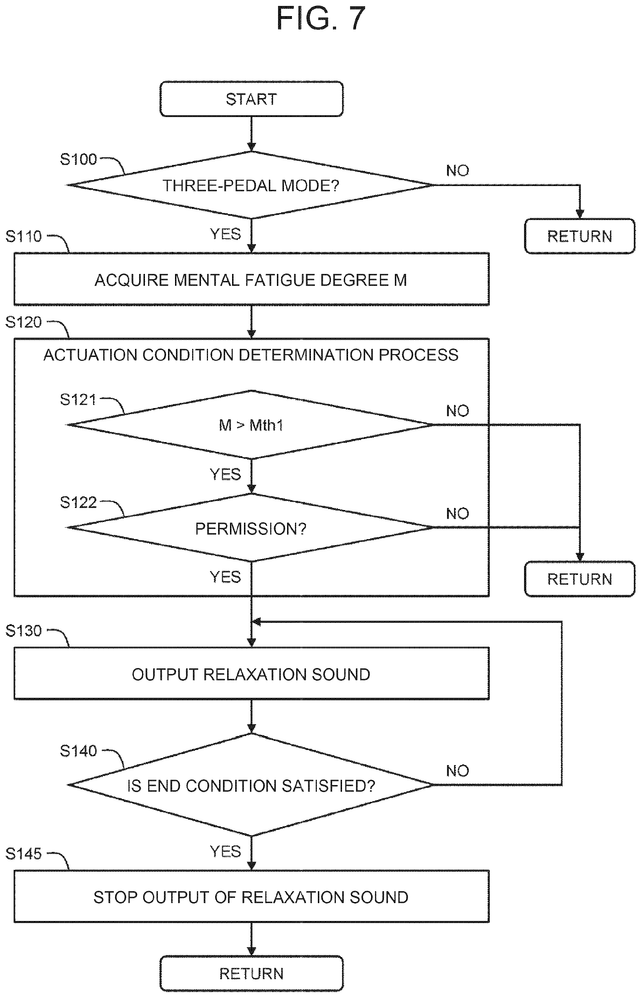

FIG. 7 is a flowchart showing an exemplary processing by the vehicle control system 100 according to the second embodiment. The same descriptions as those in the above first embodiment are omitted when appropriate. Further, a block diagram showing an exemplary functional configuration of the vehicle control system 100 according to the second embodiment is the same as that in the above first embodiment.

In step S100, the vehicle control system 100 determines whether the driving mode of the vehicle 10 (battery electric vehicle) is the three-pedal mode. In the case where the driving mode is not the three-pedal mode (step S100; No), the process in this cycle ends. On the other hand, in the case where the driving mode is the three-pedal mode (step S100; Yes), the process proceeds to step S110. In the case where the vehicle 10 is an ordinary MT vehicle, step S100 is skipped.

Step S110, step S120, step S130, and step S140 are the same as those in the first embodiment. In step S130, the relaxation sound control unit 130 acquires the relaxation sound contained in the storage device 102, and outputs the relaxation sound through the speaker 2.

2-3. Modification

The predetermined actuation condition may exclude the second condition. In that case, step S122 is skipped.

3. Third Embodiment

3-1. Outline

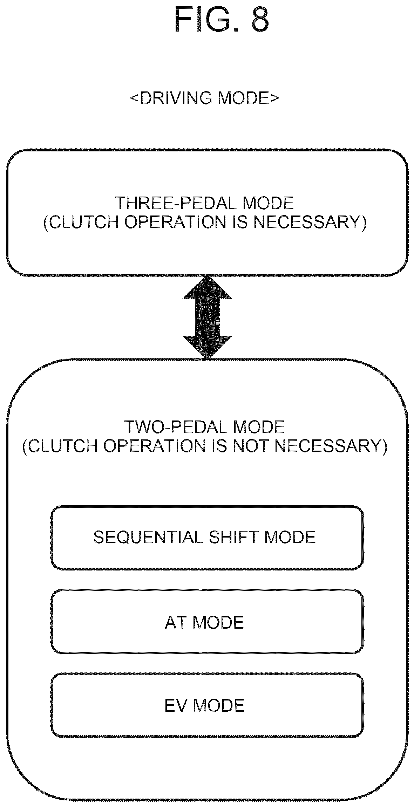

FIG. 8 is a conceptual diagram for describing an outline of a third embodiment. A vehicle 10 assumed in the third embodiment is a battery electric vehicle that uses an electric motor as a dynamic power device for traveling and that includes the pseudo clutch pedal 28 and the pseudo shift lever 27. The driving mode of the vehicle 10 (battery electric vehicle) includes the above "three-pedal mode". In the three-pedal mode, the operation of the pseudo clutch pedal 28 is necessary, and the manual gear shifting behavior and driving characteristic of the MT vehicle are simulated based on the operation of the pseudo clutch pedal 28.

The driving mode of the vehicle 10 (battery electric vehicle) further includes a "two-pedal mode" in which the operation of the pseudo clutch pedal 28 is not necessary. For example, the two-pedal mode includes an EV mode in which the vehicle 10 is driven as an ordinary battery electric vehicle. As another example, the two-pedal mode may include an AT mode in which the driving characteristic of an automatic transmission vehicle (AT vehicle) is simulated. Furthermore, as another example, the two-pedal mode may include a sequential shift mode in which the manual gear shifting behavior and driving characteristic of a sequential shift type MT vehicle are simulated. A method for realizing the sequential shift mode in the battery electric vehicle will be described later in Section 6 in detail.

During the three-pedal mode, the vehicle control system 100 (processor 101) determines whether a predetermined mode switching condition is satisfied. The predetermined mode switching condition includes at least a condition that the mental fatigue degree M of the driver exceeds a second threshold Mth2. The second threshold Mth2 may be the same as the first threshold Mth1, or may be different from the first threshold Mth1. In the case where the predetermined mode switching condition is satisfied during the three-pedal mode, the vehicle control system 100 (processor 101) switches the driving mode from the three-pedal mode in which the operation of the pseudo clutch pedal 28 is necessary to the two-pedal mode in which the operation of the pseudo clutch pedal 28 is not necessary. Thereby, the driver is freed from the operation of the pseudo clutch pedal 28, and therefore, the mental fatigue of the driver is reduced. As a result, the driver can more comfortably drive the vehicle 10.

The two-pedal mode may include the sequential shift mode and another mode (at least one of the AT mode and the EV mode). In this case, the vehicle control system 100 may switch the driving mode in the two-pedal mode, in a stepwise manner. For example, in the case where the mental fatigue degree M exceeds the second threshold Mth2, the vehicle control system 100 switches the driving mode from the three-pedal mode to the sequential shift mode. In the case where the mental fatigue degree M does not become equal to or lower than the second threshold Mth2 even when a certain time elapses from the start of the sequential shift mode, the vehicle control system 100 may switch the driving mode from the sequential shift mode to the AT mode or the EV mode.

3-2. Exemplary Functional Configuration and Exemplary Process

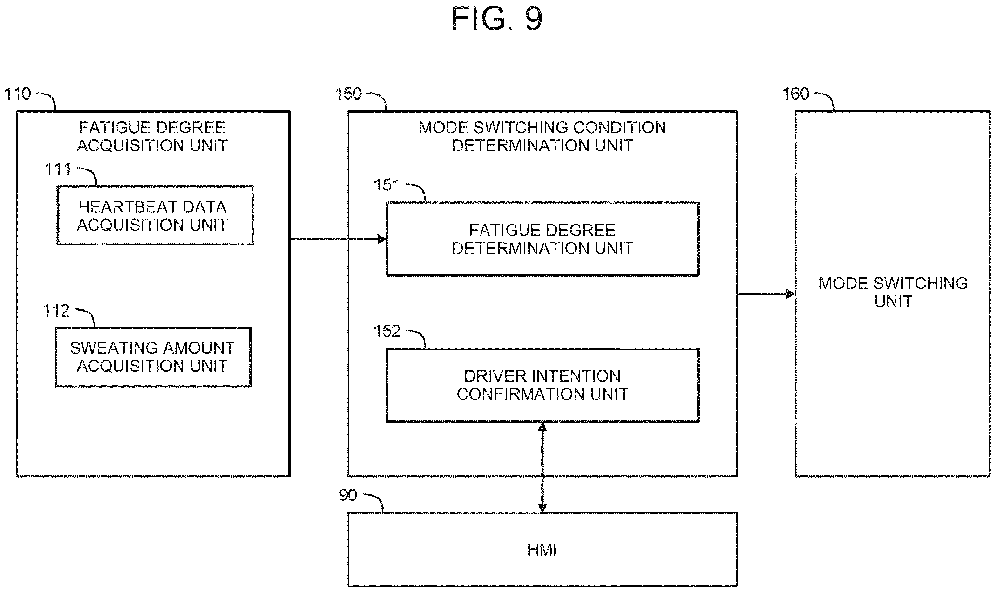

FIG. 9 is a block diagram showing an exemplary functional configuration of the vehicle control system 100 according to a third embodiment. The vehicle control system 100 includes a fatigue degree acquisition unit 110, a mode switching condition determination unit 150, and a mode switching unit 160, as functional blocks. The functional blocks may be realized by the cooperation between the processor 101 that executes the vehicle control program 105 and the storage device 102. Some of the functional blocks may be included in the management server that can communicate with the vehicle 10.

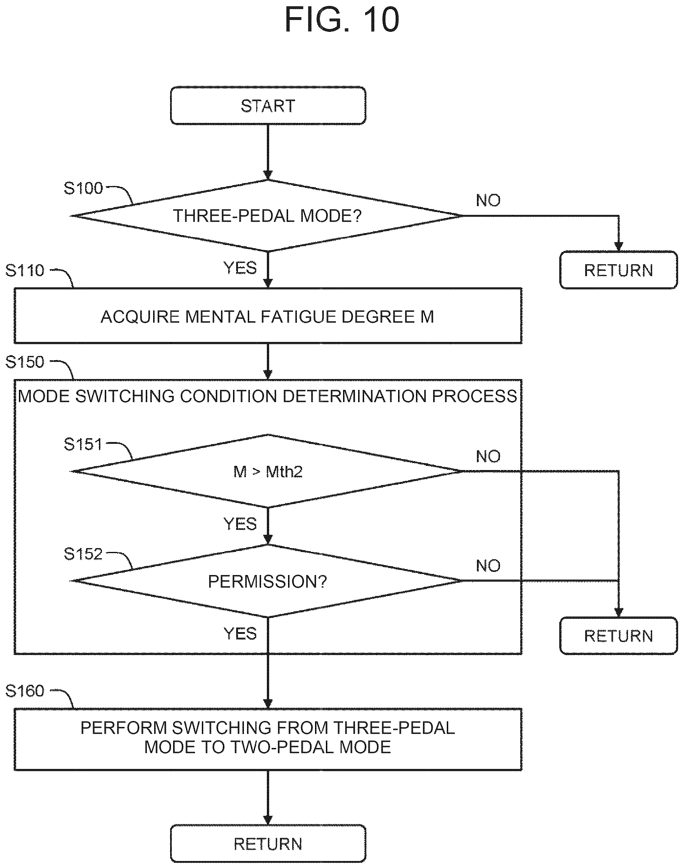

FIG. 10 is a flowchart showing an exemplary process by the vehicle control system 100 according to the third embodiment. With reference to FIG. 9 and FIG. 10, the exemplary process by the vehicle control system 100 will be described below.

In step S100, the vehicle control system 100 determines whether the driving mode of the vehicle 10 (battery electric vehicle) is the three-pedal mode. In the case where the driving mode is not the three-pedal mode (step S100; No), the process in this cycle ends. On the other hand, in the case where the driving mode is the three-pedal mode (step S100; Yes), the process proceeds to step S110.

In step S110, the fatigue degree acquisition unit 110 acquires the sensor detection information indicating the detection result of the sensor 70 that is equipped in the vehicle 10. In the case where the fatigue degree acquisition unit 110 is included in the management server, the fatigue degree acquisition unit 110 communicates with the vehicle 10, and acquires the sensor detection information. The fatigue degree acquisition unit 110 acquires the mental fatigue degree M of the driver, based on the sensor detection information.

In step S150, the mode switching condition determination unit 150 determines whether the predetermined mode switching condition is satisfied. In the case where the predetermined mode switching condition is not satisfied (step S150; No), the process in this cycle ends. On the other hand, in the case where the predetermined mode switching condition is satisfied (step S150; Yes), the process proceeds to step S160.

In the example shown in FIG. 9 and FIG. 10, the predetermined mode switching condition includes a first condition and a second condition. The first condition is that the mental fatigue degree M exceeds the second threshold Mth2. The second condition is that the driver permits the switching from the three-pedal mode to the two-pedal mode. The mode switching condition determination unit 150 includes a fatigue degree determination unit 151 and a driver intention confirmation unit 152, for determining whether the first condition and the second condition are satisfied, respectively. At least one of the fatigue degree determination unit 151 and the driver intention confirmation unit 152 may be included in the management server that can communicate with the vehicle 10.

In step S151, the fatigue degree determination unit 151 determines whether the mental fatigue degree M is higher than the second threshold Mth2, that is, whether the first condition is satisfied. In the case where the first condition is not satisfied (step S151; No), the mode switching condition is not satisfied. On the other hand, in the case where the first condition is satisfied (step S151; Yes), the process proceeds to step S152.

In step S152, the driver intention confirmation unit 152 determines whether the driver has permitted the switching from the three-pedal mode to the two-pedal mode, that is, whether the second condition is satisfied. More specifically, the driver intention confirmation unit 152 inquires of the driver whether to agree to the switching of the driving mode from the three-pedal mode to the two-pedal mode, through the output device of the HMI 90. An inquiry message may be displayed on the display, may be given from the speaker, or may be output from both the display and the speaker. In response to the inquiry message, the driver inputs "permission" or "rejection", using the input device of the HMI 90. The driver intention confirmation unit 152 can determine whether the second condition is satisfied, based on the input from the driver. In the case where the second condition is not satisfied (step S152; No), the actuation condition is not satisfied. On the other hand, in the case where the second condition is satisfied (step S152; Yes), the actuation condition is satisfied (step S150; Yes), and the process proceeds to step S160.

In step S160, the mode switching unit 160 switches the driving mode from the three-pedal mode to the two-pedal mode. When the driving mode is switched, the mode switching unit 160 may notify the driver that the driving mode is switched, through the output device of the HMI 90.

3-3. Modification

The predetermined actuation condition may exclude the second condition. In that case, step S152 is skipped.

4. Forth Embodiment

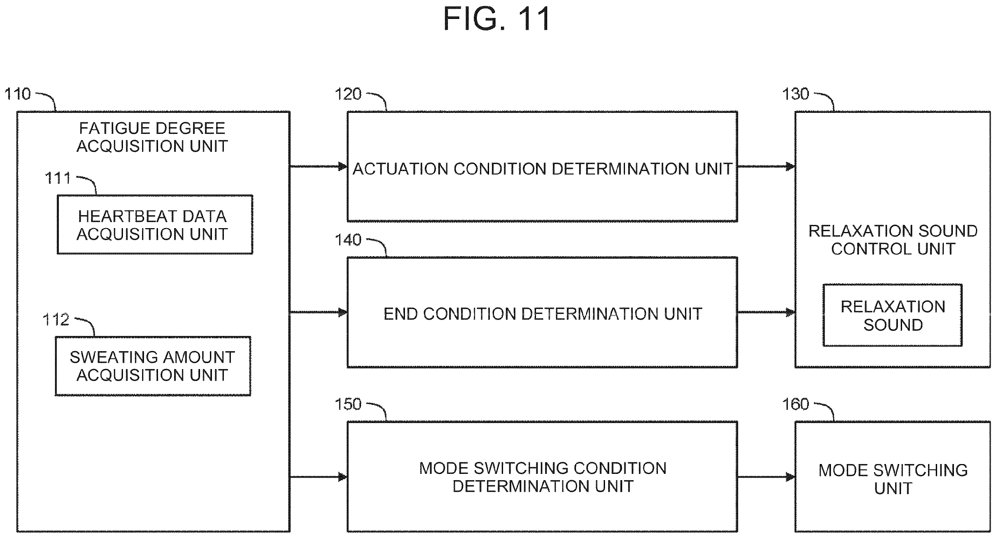

A fourth embodiment is a combination of the above second embodiment and the above third embodiment. FIG. 11 is a block diagram showing an exemplary functional configuration of a vehicle control system 100 according to the fourth embodiment. The vehicle control system 100 includes a fatigue degree acquisition unit 110, an actuation condition determination unit 120, a relaxation sound control unit 130, an end condition determination unit 140, a mode switching condition determination unit 150, and a mode switching unit 160, as functional blocks. The fatigue degree acquisition unit 110, the actuation condition determination unit 120, and the relaxation sound control unit 130 are the same as those in the above second embodiment. The mode switching condition determination unit 150 and the mode switching unit 160 are the same as those in the above third embodiment.

The actuation condition determination unit 120 and the mode switching condition determination unit 150 may behave independently from each other. That is, the vehicle control system 100 may determine whether the actuation condition is satisfied and whether the mode switching condition is satisfied, in parallel.

Alternatively, the actuation condition determination unit 120 and the mode switching condition determination unit 150 may behave in collaboration with each other. That is, the vehicle control system 100 may determine whether the actuation condition is satisfied and whether the mode switching condition is satisfied, in series.

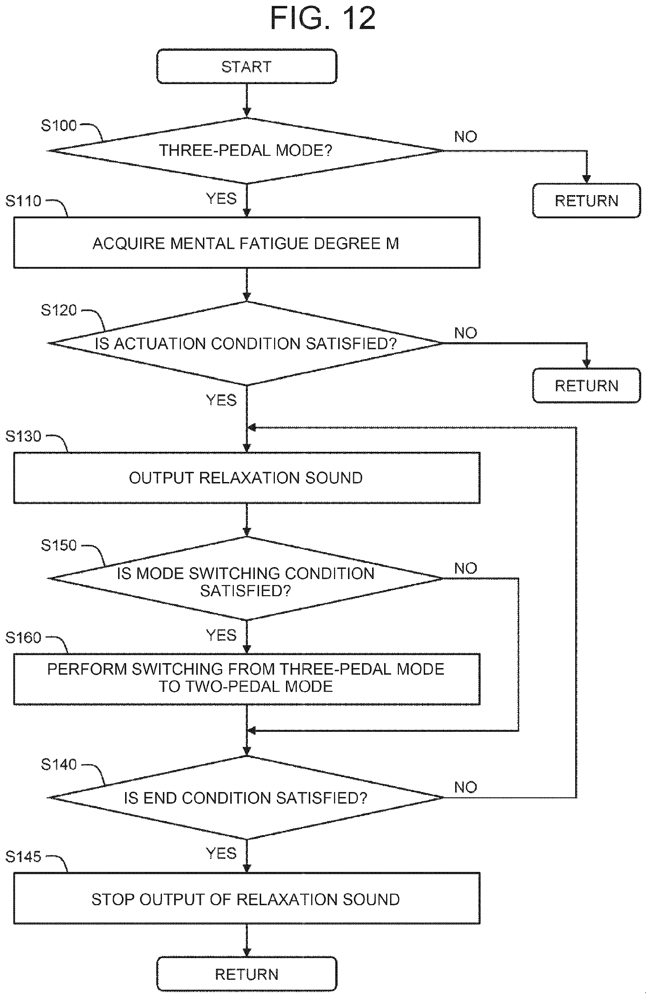

In an example shown in FIG. 12, first, the actuation condition determination unit 120 determines whether the actuation condition is satisfied. In the case where the actuation condition is satisfied (step S120; Yes), the relaxation sound control unit 130 outputs the relaxation sound (step S130). Thereafter, the mode switching condition determination unit 150 determines whether the mode switching condition is satisfied. In the case where the mode switching condition is satisfied (step S150;

Yes), the mode switching unit 160 switches the driving mode from the three-pedal mode to the two-pedal mode (step S160).

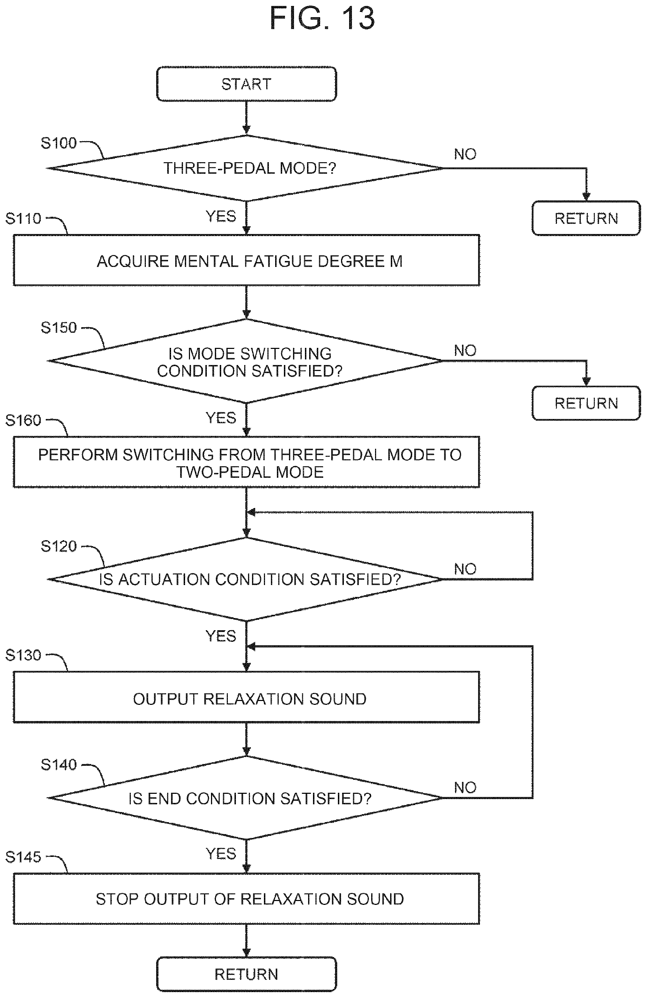

In an example shown in FIG. 13, first, the mode switching condition determination unit 150 determines whether the mode switching condition is satisfied. In the case where the mode switching condition is satisfied (step S150; Yes), the mode switching unit 160 switches the driving mode from the three-pedal mode to the two-pedal mode (step S160). Thereafter, the actuation condition determination unit 120 determines whether the actuation condition is satisfied. In the case where the actuation condition is satisfied (step S120; Yes), the relaxation sound control unit 130 outputs the relaxation sound (step S130).

## 5. Fifth Embodiment

A combination of the first embodiment and one of the second to fourth embodiments can be also adopted.

## 6. Details of MT Mode

An electric motor that is used as a dynamic power device for traveling in a general battery electric vehicle has a greatly different torque characteristic from an internal combustion engine that is used as a dynamic power device for traveling in a conventional vehicle (CV). Because of the difference in the torque characteristic of the dynamic power device, the CV needs to include a transmission, but the general battery electric vehicle does not include the transmission. Naturally, the general battery electric vehicle does not include a manual transmission (MT) in which the gear ratio is switched by the driver's manual operation. Therefore, the driving feeling is greatly different between the driving of the conventional vehicle with the MT (referred to as an MT vehicle, hereinafter) and the driving of the battery electric vehicle.

Meanwhile, in the electric motor, it is possible to control the torque relatively easily, by controlling the voltage and field that are applied. Accordingly, in the electric motor, it is possible to obtain a desired torque characteristic within the operating range of the electric motor, by executing an adequate control. With use of this feature, it is possible to simulate the torque characteristic specific to the MT vehicle, by controlling the torque of the battery electric vehicle. Further, it is possible to provide a pseudo shifter in the battery electric vehicle, such that the driver can obtain a driving feeling similar to a driving feeling for the MT vehicle. Thus, in the battery electric vehicle, it is possible to simulate the MT vehicle.

That is, the battery electric vehicle controls the output of the electric motor so as to simulate the driving characteristic (torque characteristic) specific to the MT vehicle. The driver performs a pseudo manual gear shifting operation by operating the pseudo shifter. In response to the pseudo manual gear shifting operation by the driver, the battery electric vehicle changes the driving characteristic (torque characteristic) so as to simulate the MT vehicle. Thereby, the driver of the battery electric vehicle can feel as if the driver was driving the MT vehicle. The control mode of the electric motor for simulating the driving characteristic of the MT vehicle and the manual gear shifting operation in this way is referred to as a "manual mode" or "MT mode", hereinafter.

A case where the vehicle 10 according to the present disclosure is a battery electric vehicle 10E having the MT mode will be discussed below. In the MT mode, the battery electric vehicle 10E may generate a pseudo engine sound that depends on driver's driving operation, and may output the pseudo engine sound through the speaker. Not only the driving operation of the MT vehicle but also the engine sound of the MT vehicle are reproduced, and therefore, the satisfaction level for a driver that wants reality increases. Exemplary configurations of the battery electric vehicle 10E having the MT mode will be described below. Examples of the MT mode include the "sequential shift mode" and the "three-pedal mode".

## 6-1. First Exemplary Configuration (Sequential Shift Mode)

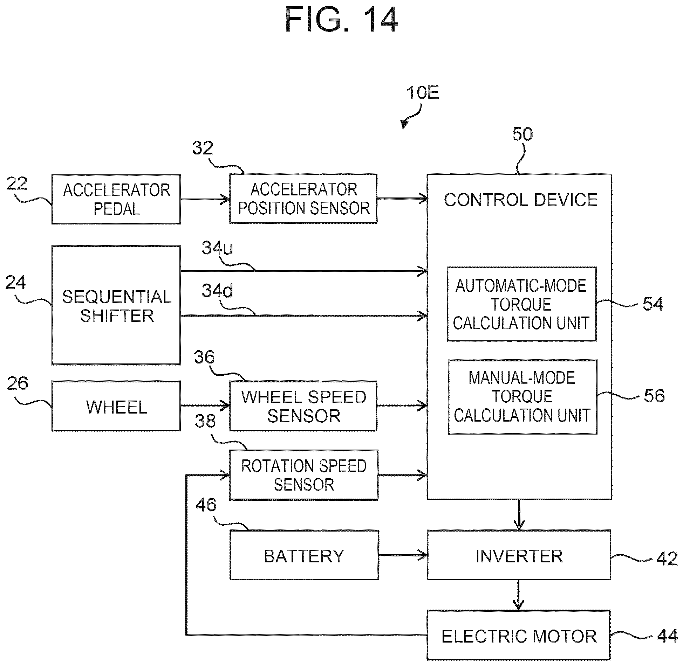

FIG. 14 is a block diagram showing a first exemplary configuration of a dynamic power control system of the battery electric vehicle 10E. The battery electric vehicle 10E includes an electric motor 44, a battery 46, and an inverter 42. The electric motor 44 is a dynamic power device for traveling. The battery 46 stores electric energy for driving the electric motor 44. That is, the battery electric vehicle 10E is a battery electric vehicle (BEV) that travels using the electric energy stored in the battery 46. The inverter 42 converts direct-current electric power that is input from the battery 46 at the time of acceleration, into drive electric power for the electric motor 44. Further, the inverter 42 converts regenerative electric power that is input from the electric motor 44 at the time of deceleration, into direct-current electric power, to charge the battery 46.

The battery electric vehicle 10E includes an accelerator pedal 22 by which the driver inputs an acceleration request for the battery electric vehicle 10E. The accelerator pedal 22 is provided with an accelerator position sensor 32 for detecting an accelerator operation amount.

The battery electric vehicle 10E includes a sequential shifter 24. The sequential shifter 24 may be a paddle-type shifter, or may be a lever-type pseudo shifter.

The paddle-type shifter is a dummy that is different from an original paddle-type shifter. The paddle-type shifter has a structure that resembles a paddle-type shifter included in a clutch pedal-less MT vehicle. The paddle-type shifter is attached to a steering wheel. The paddle-type shifter includes an upshift switch and a downshift switch that determine an operation position. The upshift switch sends an upshift signal 34u, by being pulled toward the driver, and the downshift switch sends a downshift signal 34d, by being pulled toward the driver.

Similarly to the paddle-type shifter, the lever-type pseudo shifter is a dummy that is different from an original shifter. The lever-type pseudo shifter has a structure that resembles a lever-type shifter included in the clutch pedal-less MT vehicle. The lever-type pseudo shifter outputs the upshift signal 34u when the shift lever is turned down forward, and outputs the downshift signal 34d when the shift lever is turned down rearward.

A wheel 26 of the battery electric vehicle 10E is provided with a wheel speed sensor 36. The wheel speed sensor 36 is used as a vehicle speed sensor for detecting the vehicle speed of the battery electric vehicle 10E. Further, the electric motor 44 is provided with a rotation speed sensor 38 for detecting the rotation speed of the electric motor 44.

The battery electric vehicle 10E includes a control device 50. Typically, the control device 50 is an electronic control unit (ECU) that is equipped in the battery electric vehicle 10E. The control device 50 may be a combination of a plurality of ECUs. The control device 50 includes an interface, a memory, and a processor. The interface is connected to an in-vehicle network. The memory includes a RAM that temporarily records data and a ROM that saves programs capable of being executed by the processor and a variety of data related to the programs. The program is constituted by a plurality of instructions. The processor reads programs and data from the memory, to execute the program and the data, and generates a control signal based on signals that are acquired from sensors.

For example, the control device 50 controls the electric motor 44 by the PWM control of the inverter 42. The control device 50 receives signals from the accelerator position sensor 32, the sequential shifter 24 (the upshift switch and the downshift switch in the case where the sequential shifter 24 is a paddle-type shifter), the wheel speed sensor 36, and the rotation speed sensor 38. The control device 50 processes these signals, and calculates a motor torque command value for the PWM control of the inverter 42.

The control device 50 includes an automatic mode (EV mode) and a manual mode (MT mode) as the control mode. The automatic mode is an ordinary control mode for driving the battery electric vehicle 10E as a general battery electric vehicle. In the automatic mode, by programs, the output of the electric motor 44 is continuously changed in response to the operation of the accelerator pedal 22. On the other hand, the manual mode is a control mode for driving the battery electric vehicle 10E like an MT vehicle. In the manual mode, by programs, the output characteristic of the electric motor 44 with respect to the operation of the accelerator pedal 22 is changed in response to the upshift operation and downshift operation of the sequential shifter 24. The manual mode (MT mode) corresponds to the "sequential shift mode". The switching between the automatic mode and the manual mode is possible.

The control device 50 includes an automatic-mode torque calculation unit 54 and a manual-mode torque calculation unit 56. The units 54, 56 may be ECUs that are independent from each other, or may be functions of an ECU that are obtained when programs recorded in the memory are executed by the processor.

The automatic-mode torque calculation unit 54 has a function to calculate the motor torque when the electric motor 44 is controlled in the automatic mode. In the automatic-mode torque calculation unit 54, a motor torque command map is stored. The motor torque command map is a map that determines the motor torque from the accelerator operation amount and the rotation speed of the electric motor 44. The signal of the accelerator position sensor 32 and the signal of the rotation speed sensor 38 are input to parameters of the motor torque command map. The motor torque command map outputs a motor torque corresponding to the input signals. Therefore, in the automatic mode, even when the driver operates the sequential shifter 24, the operation is not reflected in the motor torque.

The manual-mode torque calculation unit 56 includes a MT vehicle model. The MT vehicle model is a model for calculating a drive wheel torque that is obtained by the operation of the accelerator pedal 22 and the sequential shifter 24 under the assumption that the battery electric vehicle 10E is an MT vehicle.

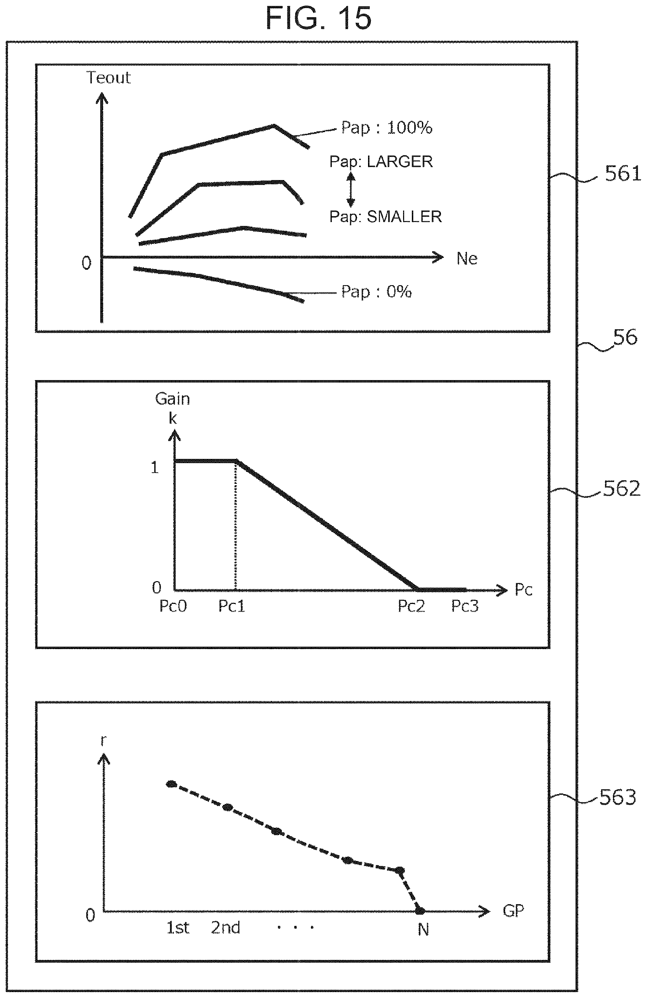

The MT vehicle model included in the manual-mode torque calculation unit 56 will be described with reference to FIG. 15. As shown in FIG. 15, the MT vehicle model includes an engine model 561, a clutch model 562, and a transmission model 563. An engine, a clutch, and a transmission that are virtually realized by the MT vehicle model are referred to as a virtual engine, a virtual clutch, and a virtual transmission, respectively. In the engine model 561, the virtual engine is modeled. In the clutch model 562, the virtual clutch is modeled. In the transmission model 563, the virtual transmission is modeled.

The engine model 561 calculates a virtual engine speed Ne and a virtual engine output torque Teout. The virtual engine speed Ne is calculated based on a rotation speed Nw of the wheel, a total speed reduction ratio R, and a slip ratio Rslip of the virtual clutch. For example, the virtual engine speed Ne is expressed as the following Expression (1)

$$\text{Expression (1): } Ne = Nw \times R / (1 - Rslip)$$

The virtual engine output torque Teout is calculated from the virtual engine speed Ne and an accelerator operation amount Pap. As shown in FIG. 15, a map that prescribes the relation of the accelerator operation amount Pap, the virtual engine speed Ne, and the virtual engine output torque Teout is used for the calculation of the virtual engine output torque Teout. In this map, the virtual engine output torque Teout with respect to the virtual engine speed Ne is given for each accelerator operation amount Pap. The torque characteristic shown in FIG. 15 can be set to a characteristic in which a gasoline engine is assumed, or can be set to a characteristic in which a diesel engine is assumed. Further, the torque characteristic can be set to a characteristic in which a naturally aspirated engine is assumed, or can be set to a characteristic in which a supercharged engine is assumed.

The clutch model 562 calculates a torque transmission gain k. The torque transmission gain k is a gain for calculating the torque transmission degree of the virtual clutch depending on a virtual clutch operation amount Pc. The virtual clutch operation amount Pc is ordinarily 0%, and is temporarily increased to 100% in conjunction with the switching of a virtual gear step of the virtual transmission. The clutch model 562 includes a map shown in FIG. 15. In this map, the torque transmission gain k is given with respect to the virtual clutch operation amount Pc. In FIG. 15, Pc0 corresponds to a position at which the virtual clutch operation amount Pc is 0%, and Pc3 corresponds to a position at which the virtual clutch operation amount Pc is 100%. A range from Pc0 to Pc1 and a range from Pc2 to Pc3 are dead zones in which the torque transmission gain k is not changed by the virtual clutch operation amount Pc. The clutch model 562 calculates a clutch output torque Tcout using the torque transmission gain k. The clutch output torque Tcout is a torque that is output from the virtual clutch. For example, the clutch output torque Tcout is given as the product of the virtual engine output torque Teout and the torque transmission gain k (Tcout = Teout $\times$ k).

Further, the clutch model 562 calculates the slip ratio Rslip. The slip ratio Rslip is used for the calculation of the virtual engine speed Ne in the engine model 561. For the calculation of the slip ratio Rslip, a map in which the slip ratio Rslip is given with respect to the virtual clutch operation amount Pc can be used, similarly to the torque transmission gain k.

The transmission model 563 calculates a gear ratio r. The gear ratio r is a gear ratio that is determined by a virtual gear step GP in the virtual transmission. In response to the upshift operation of the sequential shifter 24, the virtual gear step GP shifts up by one step. Further, in response to the downshift operation of the sequential shifter 24, the virtual gear step GP shifts down by one step. The transmission model 563 includes a map shown in FIG. 15. In this map, the gear ratio r is given with respect to the virtual gear step GP, such that the gear ratio r is lower as the virtual gear step GP is higher. The transmission model 563 calculates a transmission output torque Tgout, using the gear ratio r obtained from the map and the clutch output torque Tcout. For example, the transmission output torque Tgout is given as the product of the clutch output torque Tcout and the gear ratio r (Tgout = Tcout $\times$ r). The transmission output torque Tgout discontinuously changes in response to the switching of the gear ratio r. The discontinuous change in the transmission output torque Tgout generates gear shift shock, and the driving of a vehicle including a stepped transmission is reproduced.

The MT vehicle model calculates a drive wheel torque Tw using a predetermined speed reduction ratio rr. The speed reduction ratio rr is a fixed value that is determined by a mechanical structure from the virtual transmission to the drive wheel. The above-described total speed reduction ratio R is a value that is obtained by multiplying the speed reduction ratio rr by the gear ratio r. The MT vehicle model calculates the drive wheel torque Tw from the transmission output torque Tgout and the speed reduction ratio rr. For example, the drive wheel torque Tw is given as the product of the transmission output torque Tgout and the speed reduction ratio rr (Tw = Tgout $\times$ rr).

The control device 50 converts the drive wheel torque Tw calculated by the MT vehicle model, into a demand motor torque Tm. The demand motor torque Tm is a motor torque that is necessary for realizing the drive wheel torque Tw calculated by the MT vehicle model. For the conversion of the drive wheel torque Tw into the demand motor torque Tm, a speed reduction ratio from an output shaft of the electric motor 44 to the drive wheel is used. Moreover, in accordance with the demand motor torque Tm, the control device 50 controls the inverter 42 and controls the electric motor 44.

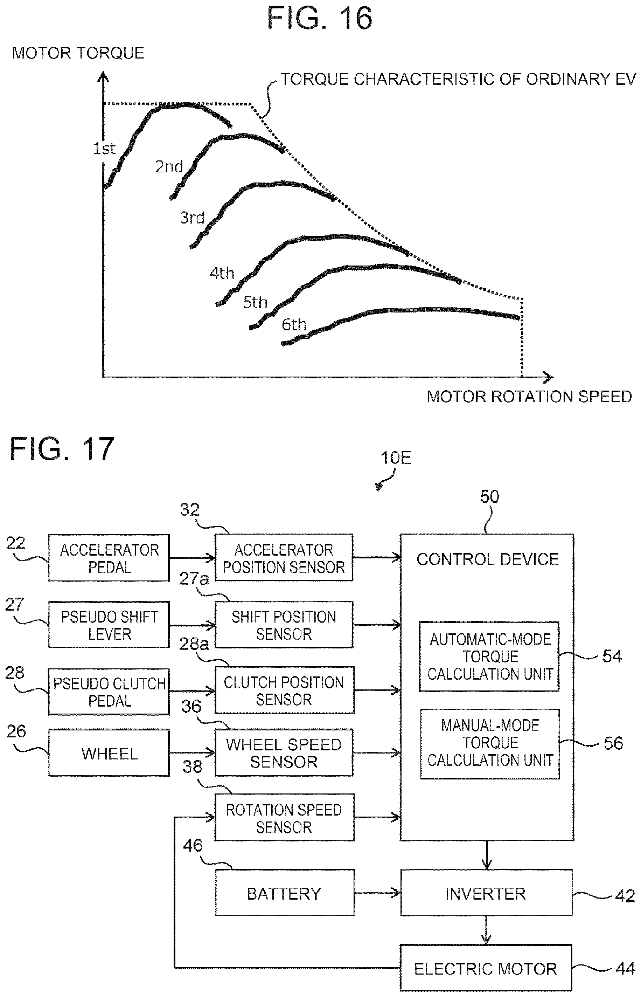

FIG. 16 is a diagram showing a torque characteristic of the electric motor 44 that is realized by the motor control using the MT vehicle model, with the comparison to a torque characteristic of the electric motor 44 that is realized by an ordinary motor control for the battery electric vehicle (EV). As shown in FIG. 16, the motor control using the MT vehicle model makes it possible to realize a torque characteristic (solid lines in the figure) that simulates the torque characteristic of the MT vehicle, depending on the virtual gear step set by the sequential shifter 24. In FIG. 16, the number of gear steps is six.

6-2. Second Exemplary Configuration (Three-Pedal Mode)

FIG. 17 is a block diagram showing a second exemplary configuration of the dynamic control system of the battery electric vehicle 10E according to the embodiment. Only different configurations from the above first exemplary configuration will be described. Specifically, in the second exemplary configuration, the battery electric vehicle 10E includes a pseudo shift lever (pseudo shift device) 27 and a pseudo clutch pedal 28, instead of the sequential shifter 24 included in the first exemplary configuration. The pseudo shift lever 27 and the pseudo clutch pedal 28 are merely dummies that are different from an original shift lever and an original clutch pedal.

The pseudo shift lever 27 has a structure that simulates the shift lever included in the MT vehicle. The disposition and operational feeling of the pseudo shift lever 27 are equivalent to those in the actual MT vehicle.

For the pseudo shift lever 27, for example, positions corresponding to gear steps: first gear, second gear, third gear, forth gear, fifth gear, sixth gear, reverse, and neutral are provided. The pseudo shift lever 27 is provided with a shift position sensor 27a that detects the gear step by discriminating the position where the pseudo shift lever 27 is placed.

The pseudo clutch pedal 28 has a structure that simulates the clutch pedal included in the MT vehicle. The disposition and operational feeling of the pseudo clutch pedal 28 are equivalent to those in the actual MT vehicle. The pseudo clutch pedal 28 is operated at the time of the operation of the pseudo shift lever 27. That is, when the driver wants to perform the setting alteration of the gear step by the pseudo shift lever 27, the driver depresses the pseudo clutch pedal 28. When the setting alteration of the gear step finishes, the driver terminates the depressing, and returns the pseudo clutch pedal 28 to the original position. The pseudo clutch pedal 28 is provided with a clutch position sensor 28a that detects the depressing amount of the pseudo clutch pedal 28.

The control device 50 receives signals from the accelerator position sensor 32, the shift position sensor 27a, the clutch position sensor 28a, the wheel speed sensor 36, and the rotation speed sensor 38. The control device 50 processes these signals, and calculates a motor torque command value for the PWM control of the inverter 42.

Similarly to the above first exemplary configuration, the control device 50 includes an automatic mode (EV mode) and a manual mode (MT mode) as the control mode. In the automatic mode, by programs, the output of the electric motor 44 is continuously changed in response to the operation of the accelerator pedal 22. On the other hand, the manual mode is a control mode for driving the battery electric vehicle 10E like an MT vehicle. In the manual mode, by programs, the output and output characteristic of the electric motor 44 with respect to the operation of the accelerator pedal 22 is changed in response to the operation of the pseudo clutch pedal 28 and the pseudo shift lever (pseudo shift device) 27. The manual mode (MT mode) corresponds to the "three-pedal mode". The switching between the automatic mode and the manual mode is possible.

The vehicle model included in the manual-mode torque calculation unit 56 is the same as the vehicle model shown in FIG. 15. However, the virtual clutch operation amount Pc is replaced with the depressing amount of the pseudo clutch pedal 28 that is detected by the clutch position sensor 28a. Further, the virtual gear step GP is determined by the position of the pseudo shift lever 27 that is detected by the shift position sensor 27a.

## Claims

1. A vehicle control system (100) configured to control a vehicle (10), the vehicle control system (100) comprising a single or plurality of processors (101), wherein:

   the single or plurality of processors (101) is configured to acquire a mental fatigue degree (M) of a driver of the vehicle (10) using at least a sensor (70) that is equipped in the vehicle (10); and
   the single or plurality of processors (101) is configured to output a relaxation sound through a speaker (2) that is equipped in the vehicle (10), when an actuation condition is satisfied, the relaxation sound being a sound for reducing the mental fatigue degree (M), the actuation condition including at least a condition that the mental fatigue degree (M) exceeds a first threshold (Mth1), wherein
   the vehicle (10) is a battery electric vehicle (10E) configured to use an electric motor (44) as a dynamic power device for traveling, and **characterized in that**:

      the vehicle (10) includes a pseudo clutch pedal (28) and a pseudo shift device (27);
      the pseudo clutch pedal (28) is operated at a time of operation of the pseudo shift device (27);
      a driving mode of the battery electric vehicle (10E) includes a three-pedal mode in which an output of the electric motor (44) with respect to operation of an accelerator pedal (22) is changed depending on the operation of the pseudo clutch pedal (28) and the operation of the pseudo shift device (27); and
      in the three-pedal mode, the single or plurality of processors (101) is configured to acquire the mental fatigue degree (M), and is configured to output the relaxation sound when the actuation condition is satisfied.

2. The vehicle control system (100) according to claim 1, wherein the actuation condition further includes a condition that the driver permits the output of the relaxation sound.

3. The vehicle control system (100) according to claim 1 or 2, wherein:

   the sensor (70) includes at least one of an electrocardiograph (71) and a sweating sensor (72), the electrocardiograph (71) and the sweating sensor (72) being equipped in a device that is used by the driver at a time of driving operation or in a wearable terminal that is worn by the driver; and
   the single or plurality of processors (101) is configured to acquire the mental fatigue degree (M), based on at least one of data about heartbeat of the driver that is detected by the electrocardiograph (71) and a sweating amount of the driver that is detected by the sweating sensor (72).

4. The vehicle control system (100) according to any one of claims 1 to 3, wherein the single or plurality of processors (101) outputs a sound in nature as the relaxation sound through the speaker (2).

5. The vehicle control system (100) according to any of claims 1 to 4, wherein the sensor (70) is equipped in the pseudo shift device (27).

6. The vehicle control system (100) according to any of claims 1 to 5, wherein:

   the driving mode of the battery electric vehicle (10E) further includes a two-pedal mode in which the operation of the pseudo clutch pedal (28) is not necessary; and
   the single or plurality of processors (101) is further configured to switch the driving mode from the three-pedal mode to the two-pedal mode, when a mode switching condition is satisfied, the mode switching condition including at least a condition that the mental fatigue degree (M) exceeds a second threshold (Mth2) during the three-pedal mode.

7. The vehicle control system (100) according to claim 6, wherein the mode switching condition further includes a condition that the driver permits the switching from the three-pedal mode to the two-pedal mode.

8. The vehicle control system (100) according to claim 6 or 7, wherein the single or plurality of processors (101) is configured to determine whether the mode switching condition is satisfied, after the actuation condition is satisfied and the relaxation sound is output.

**Patentansprüche**

1. Fahrzeugsystem (100), das dazu eingerichtet ist, ein Fahrzeug (10) zu steuern, wobei das Fahrzeugsystem (100) einen einzelnen oder eine Vielzahl von Prozessoren (101) umfasst, wobei:

   der einzelne oder die Vielzahl von Prozessoren (101) dazu eingerichtet ist bzw. sind, einen mentalen Ermüdungsgrad (M) eines Fahrers des Fahrzeugs (10) unter Verwendung mindestens eines Sensors (70), der in dem Fahrzeug (10) angeordnet ist, zu erfassen; und
der einzelne oder die Vielzahl von Prozessoren (101) dazu eingerichtet ist bzw. sind, einen Entspannungston über einen Lautsprecher (2), der in dem Fahrzeug (10) angeordnet ist, auszugeben, wenn eine Betätigungsbedingung erfüllt ist, wobei der Entspannungston ein Ton zum Verringern des mentalen Ermüdungsgrads (M) ist, wobei die Betätigungsbedingung mindestens eine Bedingung umfasst, dass der mentale Ermüdungsgrad (M) einen ersten Schwellenwert (Mth) überschreitet, wobei:
das Fahrzeug (10) ein batterieelektrisches Fahrzeug (10E) ist, das dazu eingerichtet ist, einen Elektromotor (44) als dynamische Leistungsquelle zum Fahren zu verwenden, und
das Fahrzeug (10) ein Pseudokupplungspedal (28) und eine Pseudoschaltvorrichtung (27) umfasst;
das Pseudokupplungspedal (28) zu einem Zeitpunkt des Betriebs der Pseudoschaltvorrichtung (27) betätigt wird;
ein Fahrmodus des batterieelektrischen Fahrzeugs (10E) einen Drei-Pedal-Modus umfasst, in dem eine Ausgabe des Elektromotors (44) in Bezug auf die Betätigung eines Gaspedals (22) in Abhängigkeit von der Betätigung des Pseudokupplungspedals (28) und der Betätigung der Pseudoschaltvorrichtung (27) geändert wird; und
im Drei-Pedal-Modus der einzelne oder die Vielzahl von Prozessoren (101) dazu eingerichtet ist bzw. sind, den mentalen Ermüdungsgrad (M) zu erfassen, und dazu eingerichtet ist bzw. sind, den Entspannungston auszugeben, wenn die Betätigungsbedingung erfüllt ist.

2. Fahrzeugsystem (100) nach Anspruch 1, wobei die Betätigungsbedingung ferner eine Bedingung umfasst, dass der Fahrer die Ausgabe des Entspannungstons erlaubt.

3. Fahrzeugsystem (100) nach Anspruch 1 oder 2, wobei:

   der Sensor (70) mindestens eines von einem Elektrokardiographen (71) und einem Schweißsensor (72) umfasst, wobei der Elektrokardiograph (71) und der Schweißsensor (72) in einer Vorrichtung angeordnet sind, die vom Fahrer zu einem Zeitpunkt des Fahrbetriebs verwendet wird, oder in einem tragbaren Endgerät, das vom Fahrer getragen wird; und
   der einzelne oder die Vielzahl von Prozessoren (101) dazu eingerichtet ist bzw. sind, den mentalen Ermüdungsgrad (M) basierend auf mindestens einem von Daten über einen Herzschlag des Fahrers, der durch den Elektrokardiographen (71) detektiert wird, und einer Schweißmenge des Fahrers, die durch den Schweißsensor (72) detektiert wird, zu erfassen.

4. Fahrzeugsystem (100) nach einem der Ansprüche 1 bis 3, wobei der einzelne oder die Vielzahl von Prozessoren (101) einen Naturton als den Entspannungston über den Lautsprecher (2) ausgibt bzw. ausgeben.

5. Fahrzeugsystem (100) nach einem der Ansprüche 1 bis 4, wobei der Sensor (70) in der Pseudoschaltvorrichtung (27) angeordnet ist.

6. Fahrzeugsystem (100) nach einem der Ansprüche 1 bis 5, wobei:

der Fahrmodus des batterieelektrischen Fahrzeugs (10E) ferner einen Zwei-Pedal-Modus umfasst, in dem die Betätigung des Pseudokupplungspedals (28) nicht erforderlich ist; und der einzelne oder die Vielzahl von Prozessoren (101) ferner dazu eingerichtet ist bzw. sind, den Fahrmodus vom Drei-Pedal-Modus in den Zwei-Pedal-Modus umzuschalten, wenn eine Modusumschaltbedingung erfüllt ist, wobei die Modusumschaltbedingung mindestens eine Bedingung umfasst, dass der mentale Ermüdungsgrad (M) während des Drei-Pedal-Modus einen zweiten Schwellenwert (Mth2) überschreitet.

7. Fahrzeugsystem (100) nach Anspruch 6, wobei die Modusumschaltbedingung ferner eine Bedingung umfasst, dass der Fahrer das Umschalten vom Drei-Pedal-Modus in den Zwei-Pedal-Modus erlaubt.

8. Fahrzeugsystem (100) nach Anspruch 6 oder 7, wobei der einzelne oder die Vielzahl von Prozessoren (101) dazu eingerichtet ist bzw. sind, zu bestimmen, ob die Modusumschaltbedingung erfüllt ist, nachdem die Betätigungsbedingung erfüllt ist und der Entspannungston ausgegeben wird.

## Revendications

1. Système de commande de véhicule (100) configuré pour commander un véhicule (10), le système de commande de véhicule (100) comprenant un unique processeur ou une pluralité de processeurs (101), dans lequel :

l'unique processeur ou la pluralité de processeurs (101) est configuré pour acquérir un degré de fatigue mentale (M) d'un conducteur du véhicule (10) en utilisant au moins un capteur (70) qui est monté dans le véhicule (10) ; et l'unique processeur ou la pluralité de processeurs (101) est configuré pour délivrer un son de relaxation par l'intermédiaire d'un haut-parleur (2) qui est monté dans le véhicule (10), quand une condition d'actionnement est satisfaite, le son de relaxation étant un son destiné à réduire le degré de fatigue mentale (M), la condition d'actionnement comprenant au moins une condition selon laquelle le degré de fatigue mentale (M) dépasse un premier seuil (Mth1), dans lequel

le véhicule (10) est un véhicule électrique à batterie (10E) configuré pour utiliser un moteur électrique (44) comme dispositif de puissance dynamique pour le déplacement, et **caractérisé en ce que** :

le véhicule (10) comprend une pédale de pseudo embrayage (28) et un pseudo dispositif de changement de vitesse (27) ; la pédale de pseudo embrayage (28) est actionnée au moment de l'actionnement du pseudo dispositif de changement de vitesse (27) ; un mode de conduite du véhicule électrique à batterie (10E) comprend un mode à trois pédales dans lequel une sortie du moteur électrique (44) par rapport à un actionnement d'une pédale d'accélérateur (22) est changée en fonction de l'actionnement de la pédale de pseudo embrayage (28) et de l'actionnement du pseudo dispositif de changement de vitesse (27) ; et dans le mode à trois pédales, l'unique processeur ou la pluralité de processeurs (101) est configuré pour acquérir le degré de fatigue mentale (M), et est configuré pour délivrer le son de relaxation quand la condition d'actionnement est satisfaite.

2. Système de commande de véhicule (100) selon la revendication 1, dans lequel la condition d'actionnement comprend en outre une condition selon laquelle le conducteur permet la sortie du son de relaxation.

3. Système de commande de véhicule (100) selon la revendication 1 ou 2, dans lequel :

le capteur (70) comprend au moins un d'un électrocardiographe (71) et d'un capteur de transpiration (72), l'électrocardiographe (71) et le capteur de transpiration (72) étant montés dans un dispositif qui est utilisé par le conducteur au moment de l'opération de conduite ou dans un terminal portable qui est porté par le conducteur ; et

l'unique processeur ou la pluralité de processeurs (101) est configuré pour acquérir le degré de fatigue mentale (M), sur la base de au moins une de données concernant le battement de cœur du conducteur qui est détecté par l'électrocardiographe (71) et une quantité de transpiration du conducteur qui est détectée par le capteur de transpiration (72).

4. Système de commande de véhicule (100) selon l'une quelconque des revendications 1 à 3, dans lequel l'unique processeur ou la pluralité de processeurs (101) délivre un son de nature comme son de relaxation par l'intermédiaire du haut-parleur (2).

5. Système de commande de véhicule (100) selon l'une quelconque des revendications 1 à 4, dans lequel le capteur (70) est monté dans le pseudo dispositif de changement de vitesse (27).

6. Système de commande de véhicule (100) selon l'une quelconque des revendications 1 à 5, dans lequel :

le mode de conduite du véhicule électrique à batterie (10E) comprend en outre un mode à deux pédales dans lequel l'actionnement de la pédale de pseudo embrayage (28) n'est pas nécessaire ; et
l'unique processeur ou la pluralité de processeurs (101) est en outre configuré pour commuter le mode de conduite du mode à trois pédales au mode à deux pédales, quand une condition de commutation de mode est satisfaite, la condition de commutation de mode comprenant au moins une condition selon laquelle le degré de fatigue mentale (M) dépasse un deuxième seuil (Mth2) pendant le mode à trois pédales.

7. Système de commande de véhicule (100) selon la revendication 6, dans lequel la condition de commutation de mode comprend en outre une condition selon laquelle le conducteur permet la commutation du mode à trois pédales au mode à deux pédales.

8. Système de commande de véhicule (100) selon la revendication 6 ou 7, dans lequel l'unique processeur ou la pluralité de processeurs (101) est configuré pour déterminer si la condition de commutation de mode est satisfaite, une fois que la condition d'actionnement est satisfaite et le son de relaxation est délivré.

# FIG. 1

# FIG. 2

# FIG. 3A

VOLTAGE

HEARTBEAT
INTERVAL (RRI)

TIME

# FIG. 3B

y

RRI(n+1)

AREA S

RRI(n)

x

# FIG. 3C

SWEATING AMOUNT

CONGESTION

VEHICLE CUTTING-IN

TIME

# FIG. 4

110 FATIGUE DEGREE ACQUISITION UNIT

111 HEARTBEAT DATA ACQUISITION UNIT

112 SWEATING AMOUNT ACQUISITION UNIT

120 ACTUATION CONDITION DETERMINATION UNIT

121 FATIGUE DEGREE DETERMINATION UNIT

122 DRIVER INTENTION CONFIRMATION UNIT

90 HMI

140 END CONDITION DETERMINATION UNIT

130 RELAXATION SOUND CONTROL UNIT

RELAXATION SOUND

EP 4 566 521 B1

# FIG. 5

```
                          ┌──────────────┐
                          │    START     │
                          └──────────────┘
                                 │
                                 ▼
S110 ─┐  ┌────────────────────────────────────────────┐
      └──│     ACQUIRE MENTAL FATIGUE DEGREE M         │
         └────────────────────────────────────────────┘
                                 │
                                 ▼
S120 ─┐  ┌────────────────────────────────────────────┐
      └──│  ACTUATION CONDITION DETERMINATION PROCESS  │
```

S121 — M > Mth1  NO

YES

S122 — PERMISSION?  NO

YES

RETURN

```
S130 ─┐  ┌────────────────────────────────────────────┐
      └──│          OUTPUT RELAXATION SOUND            │
         └────────────────────────────────────────────┘
```

S140 — IS END CONDITION SATISFIED?  NO

YES

```
S145 ─┐  ┌────────────────────────────────────────────┐
      └──│      STOP OUTPUT OF RELAXATION SOUND        │
         └────────────────────────────────────────────┘
```

RETURN

# FIG. 6

THREE-PEDAL MODE

# FIG. 7

```
                    START

S100      THREE-PEDAL MODE?              NO
                                              RETURN
             YES

S110
        ACQUIRE MENTAL FATIGUE DEGREE M

S120
        ACTUATION CONDITION DETERMINATION PROCESS

    S121                                   NO
                    M > Mth1

                 YES

    S122                                   NO
                  PERMISSION?
                                              RETURN
                 YES

S130
           OUTPUT RELAXATION SOUND

S140                                       NO
        IS END CONDITION SATISFIED?

             YES

S145
        STOP OUTPUT OF RELAXATION SOUND

                  RETURN
```

# FIG. 8

<DRIVING MODE>

THREE-PEDAL MODE
(CLUTCH OPERATION IS NECESSARY)

TWO-PEDAL MODE
(CLUTCH OPERATION IS NOT NECESSARY)

SEQUENTIAL SHIFT MODE

AT MODE

EV MODE

# FIG. 9

# FIG. 10

START

S100 — THREE-PEDAL MODE? — NO → RETURN

YES

S110 — ACQUIRE MENTAL FATIGUE DEGREE M

S150 — MODE SWITCHING CONDITION DETERMINATION PROCESS

S151 — $M > Mth2$ — NO

YES

S152 — PERMISSION? — NO → RETURN

YES

S160 — PERFORM SWITCHING FROM THREE-PEDAL MODE TO TWO-PEDAL MODE

RETURN

# FIG. 11

110 — FATIGUE DEGREE ACQUISITION UNIT

111 — HEARTBEAT DATA ACQUISITION UNIT

112 — SWEATING AMOUNT ACQUISITION UNIT

120 — ACTUATION CONDITION DETERMINATION UNIT

140 — END CONDITION DETERMINATION UNIT

150 — MODE SWITCHING CONDITION DETERMINATION UNIT

130 — RELAXATION SOUND CONTROL UNIT

RELAXATION SOUND

160 — MODE SWITCHING UNIT

EP 4 566 521 B1

# FIG. 12

```
                    START

S100        THREE-PEDAL MODE?        NO → RETURN

            YES

S110   ACQUIRE MENTAL FATIGUE DEGREE M

S120   IS ACTUATION CONDITION SATISFIED?   NO → RETURN

            YES

S130   OUTPUT RELAXATION SOUND

S150   IS MODE SWITCHING CONDITION SATISFIED?   NO

            YES

S160   PERFORM SWITCHING FROM THREE-PEDAL MODE
       TO TWO-PEDAL MODE

S140   IS END CONDITION SATISFIED?   NO

            YES

S145   STOP OUTPUT OF RELAXATION SOUND

                    RETURN
```

# FIG. 13

```
                          ┌─────────────────┐
                          │      START      │
                          └─────────────────┘
                                   │
         S100                      ▼
            ◄──────────────────────────────────────────────────►   NO
                    THREE-PEDAL MODE?                    ──────────────►  ┌──────────┐
                                                                          │  RETURN  │
         S110              YES                                            └──────────┘
            ┌──────────────────────────────────────────────┐
            │      ACQUIRE MENTAL FATIGUE DEGREE M          │
            └──────────────────────────────────────────────┘
                                   │
         S150                      ▼
            ◄──────────────────────────────────────────────────►   NO
                    IS MODE SWITCHING
                    CONDITION SATISFIED?                    ──────────────►  ┌──────────┐
                                                                            │  RETURN  │
         S160              YES                                              └──────────┘
            ┌──────────────────────────────────────────────┐
            │  PERFORM SWITCHING FROM THREE-PEDAL MODE TO   │
            │              TWO-PEDAL MODE                   │
            └──────────────────────────────────────────────┘
                                   │
         S120                      ▼
            ◄──────────────────────────────────────────────────►   NO
                    IS ACTUATION CONDITION SATISFIED?      ───────────────┐
                                   │                                      │
         S130              YES                                            │
            ┌──────────────────────────────────────────────┐             │
            │          OUTPUT RELAXATION SOUND             │◄────────────┘
            └──────────────────────────────────────────────┘
                                   │
         S140                      ▼
            ◄──────────────────────────────────────────────────►   NO
                    IS END CONDITION SATISFIED?            ───────────────┘
                                   │
         S145              YES
            ┌──────────────────────────────────────────────┐
            │      STOP OUTPUT OF RELAXATION SOUND          │
            └──────────────────────────────────────────────┘
                                   │
                                   ▼
                          ┌─────────────────┐
                          │     RETURN      │
                          └─────────────────┘
```

# FIG. 14

10E

| | |
|---|---|
| 22 ACCELERATOR PEDAL | 32 ACCELERATOR POSITION SENSOR |
| 24 SEQUENTIAL SHIFTER | 34u / 34d |
| 26 WHEEL | 36 WHEEL SPEED SENSOR |
| 38 ROTATION SPEED SENSOR | |
| 46 BATTERY | 42 INVERTER |
| | 44 ELECTRIC MOTOR |

50 CONTROL DEVICE

54 AUTOMATIC-MODE TORQUE CALCULATION UNIT

56 MANUAL-MODE TORQUE CALCULATION UNIT

# FIG. 15

# FIG. 16

MOTOR TORQUE

TORQUE CHARACTERISTIC OF ORDINARY EV

1st

2nd

3rd

4th

5th

6th

MOTOR ROTATION SPEED

# FIG. 17

10E

| 22 ACCELERATOR PEDAL | 32 ACCELERATOR POSITION SENSOR | 50 CONTROL DEVICE |
|---|---|---|

27a

| 27 PSEUDO SHIFT LEVER | SHIFT POSITION SENSOR | AUTOMATIC-MODE TORQUE CALCULATION UNIT ~ 54 |
|---|---|---|

28a

| 28 PSEUDO CLUTCH PEDAL | CLUTCH POSITION SENSOR | MANUAL-MODE TORQUE CALCULATION UNIT ~ 56 |
|---|---|---|

36

| 26 WHEEL | WHEEL SPEED SENSOR | |
|---|---|---|

38

ROTATION SPEED SENSOR

46

| BATTERY | INVERTER ~ 42 |
|---|---|

ELECTRIC MOTOR ~ 44

**EP 4 566 521 B1**

**REFERENCES CITED IN THE DESCRIPTION**

**Patent documents cited in the description**

- JP 6787507 B
- JP 2023077193 A
- US 2020383580 A1
- US 2022041070 A1
- US 2021229550 A1